# EUROPEAN PATENT APPLICATION

(11) **EP 3 130 901 A1**
(43) Date of publication of application: **15.02.2017**
(21) Application number: 15775934.1
(22) Date of filing: 10.03.2015
(51) Int. Cl.: G01M 3/28, A61B 1/00

(54) **AUTOMATIC ENDOSCOPE LEAKAGE INSPECTION APPARATUS AND INSPECTION METHOD THEREOF**

(30) Priority: 07.04.2014 KR 20140040942
(71) Applicant: Diabell Co., Ltd., Anyang-si, Gyeonggi-do 430-857 (KR); Choi, Dong Yeol, Seongnam-si, Gyeonggi-do 461-809 (KR); Park, Jeong Hui, Seongnam-si, Gyeonggi-do 463-876 (KR)
(72) Inventor: CHOI, Dong Yeol, Gyeonggi-do 461-809 (KR); PARK, Jeong Hui, Gyeonggi-do 463-876 (KR); KIM, Sam Jong, Anyang-si Gyeonggi-do 431-759 (KR)
(74) Representative: Garavelli, Paolo
(86) International application number: PCT/KR2015/002283
(87) International publication number: WO 2015/156505

(57) **Abstract**

The present invention relates to an automatic endoscope leakage inspection apparatus and an inspection method thereof. The endoscope includes RFID tags having unique identification information depending on the types of various endoscopes, and the automatic endoscope leakage inspection apparatus includes an RFID module for reading the unique identification information of the endoscope from the RFID tags. The automatic endoscope leakage inspection apparatus manages the endoscope information depending on the types of various endoscopes, reads the unique identification information on the endoscope using an RF communication network, selects one piece of information on the endoscope corresponding to the read unique identification information and automatically processes the leakage inspection by the endoscope. The present invention can facilitate the leakage inspection by the endoscope and improve the accuracy of the leakage inspection by managing the data for each type of the various endoscopes using an RFID communication network.

## Description

### Technical Field

The present invention relates to an automatic endoscope leakage inspection apparatus, and in particular to an automatic endoscope leakage inspection apparatus and an inspection method thereof, wherein the endoscope information on various endoscopes are managed by kinds, and an unique identification number of a predetermined endoscope is read out using a RF communication network, and one endoscope information corresponding to the read unique identification information is selected, thus automatically processing a leakage inspection of the endoscope.

### Background Art

The endoscopeis referred to a device which is configured to inspect an internal body or surgically operate or is inserted into the inside of an internal organ and observe a corresponding internal organ, for example, a bronchus, a throat, a stomach, a heart, a large intestine, etc. which cannot be visually checked. There are various kinds of endoscopes which may be categorized, for example, by manufacturers, use purposes, sizes, etc. The endoscope is designed to observe a predetermined internal organ using a small-sized camera, for example, a CCD sensor, etc. For example, the endoscope is equipped with a scope unit at an end thereof, which is inserted into an internal body. The scope unit is formed of a CCD camera to obtain images, an angle unit for driving the motion of the CCD camera, and a light source which emits light to a predetermined subject.

For this, the endoscope is provided with a plurality of tubular passages through which a medicinal fluid, a gas, etc. are injected. Moreover, the endoscope is provided with a tubular passage into which a plurality of cables are inserted to control the motions of a small-sized camera or emit light.

Before the start of the operation, this endoscope necessarily needs a procedure to inspect any damage to the tubular passage. If a predetermined portion of the tubular passage is damaged, the scope unit may be exposed to a medicinal fluid or a gas which is being supplied to the tubular passage. In this case, it may cause a problem with the endoscope. In worse case, the whole components of the endoscope should be discarded, thus causing a critical financial problem. For this reason, the leakage inspection should be carried out before the operation.

In many typical hospitals, an endoscope leakage inspection is carried out using an endoscope washer which is configured to wash the endoscope before the endoscope is used for an operation, and if no abnormality, the endoscope is used for the operation. After the operation is finished, the leakage inspection should be carried out. If no abnormality, the washing and disinfection are carried out for the following reason. If the operation, washing or disinfection is carried out in a state where the endoscope has a leakage, water or mucus may enter the inside of the endoscope due to the leakage, thus causing a problem withvarious electronic components disposed inside of the endoscope, for example, a CCD, a camera, an angle adjusting device, a lighting device, etc.

The conventional endoscope leakage inspection device, however, may need a long inspection time and may be hard to locate the position of the leakage and may need a very professional skill, for which the reliability of the inspection may be degraded.

In order to resolve the aforementioned problems, the Korean patent registration number 10-1052893 (the laid-open date is July 29, 2011) filed by the same applicant as the present invention describes the invention entitled "an endoscope leakage inspection apparatus and a leakage inspection method using the same", wherein the endoscope leakage inspection apparatus is installed at the endoscope using a connector and is able to easily, accurately and fast inspect any leakage of the endoscope in an automatic manner.

In the aforementioned endoscope leakage inspection apparatus, since various kinds of the endoscope are not separately managed, it is impossible to carry out an accurate leakage inspection by kinds of the endoscopes. More specifically, since the inside tubular passages of the endoscopes may have different lengths and thicknesses based on the manufacturers and use purposes, the inspection conditions may be different during the endoscope leakage inspection. For this reason, the inspections should be carried out after each inspection condition has been manually carried out, which may cause an inaccurate inspection.

### Disclosure of Invention

Accordingly, it is an object of the present invention to provide an automatic endoscope leakage inspection apparatus and an inspection method thereof which may make easier the leakage inspection based on the kinds of various endoscopes.

It is another object of the present invention to provide an automatic endoscope leakage inspection apparatus and an inspection method thereof which are able to automatically recognize an endoscope and the kinds of the endoscopes using a wireless communication and are able to automatically inspect an accurate leakage state through the same.

It is further another object of the present invention to provide an automatic endoscope leakage inspection apparatus and an inspection method thereof which allow for an easier engagement and carriage in response to the kinds of various endoscopes, wherein the operations thereof are easy.

The automatic endoscope leakage inspection apparatus according to the present invention is referred to managing the data by kinds of various endoscopes. This automatic endoscope leakage inspection apparatus is able to automatically recognize the mounted endoscope through a wireless communication, thus automatically processing the leakage inspection.

To achieve the above objects, there is provided an automatic endoscope leakage inspection apparatus, which may include, but is not limited to, an RFID tag which is provided at an endoscope having an inside tubular passage and has a unique identification information on the endoscope; an RFID module which is able to read out the unique identification information on the endoscope, which will be subjected to a leakage inspection, from the RFID tag;an inspection module which is formed of an air supply unit configured to supply the air of a constant pressure to the inside tubular passage of the endoscope, a pressure sensor configured to measure a pressure change in the inside tubular passage of the endoscope, and a relief valve configured to maintain a stable pressurization on the inside tubular passage of the endoscope, wherein the inspection module is able to supply, for a predetermined time period, the air of a set pressurization pressure to the inside tubular passage of the endoscope and is able to detect the pressure change; anda controller which is configured to set and store a plurality of endoscope information corresponding to the endoscopes of various kinds in the inside thereof, control for the RFID module to read out the unique identification information from the RFID tag, set a pressurization pressure, a pressurization time, a volume value and a stabilization time by kinds of the endoscopes in such a way to use the endoscope information corresponding to the unique identification information read out using the RFID module, supply the air of a constant pressure to the inside tubular passage formed inside of the endoscope, monitor a pressure change in the inside of the endoscope via the same, control the inspection module for the leakage of the inside tubular passage of the endoscope to be automatically inspected, and determine the leakage inspection state of the endoscope in response to the pressure change detected by the inspection module.

According to an embodiment of the present invention, the automatic. endoscope leakage inspection apparatus further include a display unit which is able to display a leakage inspection state of the endoscope determined by the controller.

According to another embodiment of the present invention, the inspection module may include an air supply unit for supplying the air of a constant pressure to the inside tubular passage of the endoscope, a pressure sensor for measuring a pressure change in the inside tubular passage of the endoscope, and a relief valve for maintaining a stable pressurization in the inside tubular passage of the endoscope.

According to another embodiment of the present invention, the controller is able to manually select the endoscope information with respect to the endoscope which will be subjected to a leakage inspection.

According to a feature of the present invention, there is provided an inspection method of an automatic endoscope leakage inspection apparatus which is able to automatically carry out a leakage inspection of the endoscope provided with an RFID tag containing a unique identification information.

To achieve the above objects, there is provided an inspection method of an automatic endoscope leakage inspection apparatus which is able to automatically carry out a leakage inspection of the endoscope formed of an RFID tag including a unique identification information, which may include, but is not limited to, setting and storing an endoscope information with respect to each of a plurality of endoscopes based on various kinds thereof at the automatic endoscope leakage inspection apparatus;selecting an inspection mode on if the leakage inspection is proceeded in the automatic mode or the manual mode with respect to an endoscope when the endoscope has been mounted on the automatic endoscope leakage inspection apparatus;reading out the RFID tag of the mounted endoscope using an RFID module of the automatic endoscope leakage inspection apparatus when the inspection mode is selected as the automatic mode;automatically selecting the endoscope information corresponding to the unique identification information read out from the RFID tag; andsetting a pressurization pressure, a pressurization time, a volume value and a stabilization time based on the kinds of the endoscopes in response to the endoscope information when the endoscope information is automatically selected, and pressurizing the air to an inside tubular passage of the endoscope, and carrying out an automatic leakage inspection.

According to an embodiment of the present invention, the endoscope information may include at least one of the kinds of an endoscope, a manufacturer, a serial number, a pressurization pressure, a pressurization time, a stabilization time, a volume valve and a reference pressure.

According to another embodiment of the present invention, the inspection method is referred to manually selecting the kinds of the endoscope which will be subjected to the leakage inspection if the inspection mode is selected as the manual mode, and automatically selecting the endoscope information corresponding to the kinds of the selected endoscope.

According to another embodiment of the present invention, the inspection method may further include displaying a result of the leakage inspection which has been carried out.

### Advantageous Effects of the Invention

The automatic endoscope leakage inspection apparatus according to the present invention is able to easily carry out the leakage inspection of the endoscope in such a way to manage the data by kinds of various endoscopes, thus facilitating the leakage inspection of the endoscope and enhancing the accuracy of the leakage inspection.

Moreover, the automatic endoscope leakage inspection apparatus according to the present invention is able to automatically carry out the leakage inspection by kinds of various endoscopes in such a way to read out the unique identification information of the endoscope which will be used to inspect the leakage using a wireless communication network and use one endoscope information corresponding to the unique identification information of the read endoscope.

### Brief Description of Drawings

Figure 1 is a perspective view illustrating a connection configuration of an automatic endoscope leakage inspection apparatus according to the present invention.
Figure 2 is a block diagram illustrating a configuration of an automatic endoscope leakage inspection apparatus in Figure 1.
Figure 3 is a view illustrating a configuration of an endoscope information set in a memory in Figure 2.
Figures 4 to 9 are views illustrating a setting procedure and an inspection result based on the kinds of the endoscopes are displayed on a display unit in Figure 1.
Figure 10 is a flow chart illustrating an inspection procedure of an automatic endoscope leakage inspection apparatus according to the present invention.
Figure 11 is a flow chart illustrating an automatic leakage inspection procedure in Figure 10.

### Best Modes for carrying out the invention

The embodiments of the present invention may be modified into various forms, and it should not be interpreted that the scope of the present invention is limited by the embodiments which will be described below. The present invention embodiments are provided to describe the present invention more completely to a person having ordinary skill in the art. The shapes, etc. of the components in the drawings may be a little exaggerated for the sake of a clarified description.

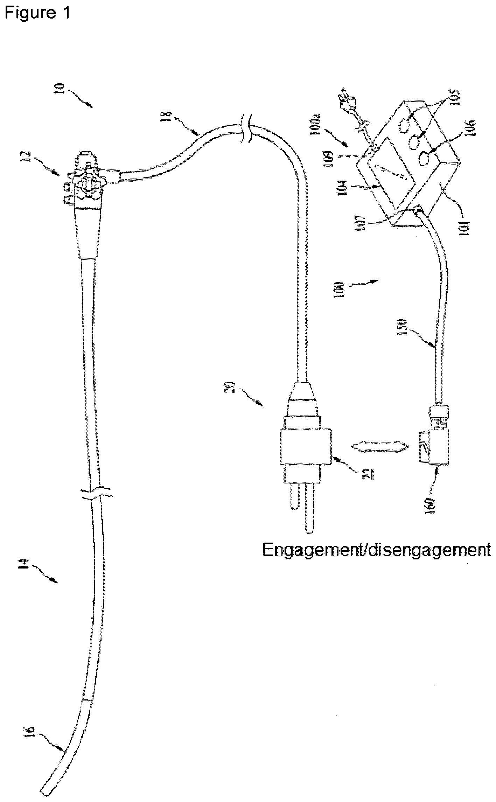

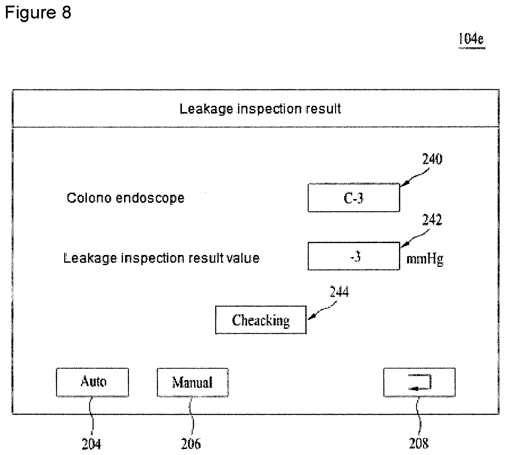

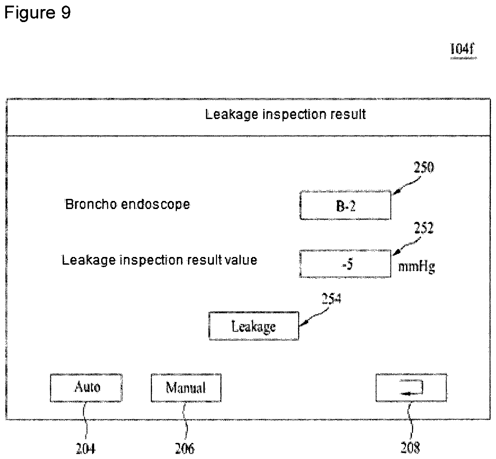

The embodiments of the present invention will be described with reference to Figures 1 to 11.

The present invention is referred to an invention which is able to manage the data by kinds of endoscopes and automatically process the leakage inspections of the endoscope through the same, which is improved from the endoscope leakage inspection apparatus and leakage inspection method using the same of the Korean patent registration number 10-1052893 (July 29, 2011) filed by the same applicant as the present invention. So, the improved technical features of the automatic endoscope leakage inspection apparatus of the present invention will be intensively described based on the configuration and operation of the patent-registered endoscope leakage inspection apparatus.

Figure 1 is a perspective view illustrating a configuration of an automatic endoscope leakage inspection apparatus which is equipped with an endoscope according to the embodiment of the present invention.

Referring to Figure 1, the automatic endoscope leakage inspection apparatus 100 according to the present invention is mounted at the endoscope 10 so as to automatically inspect the leakage state of the endoscope 10 and is able to recognize a unique identification information of the endoscope 10 through a wireless communication with the mounted endoscope 10 and automatically inspect the leakage state using the endoscope information set in accordance with a unique identification information corresponding to the endoscope 10 through the same.

The automatic endoscope leakage inspection apparatus 100 according to the present invention may automatically inspect the leakage state of the endoscope 10 in such a way to supply and pressurize the air at a predetermined pressure set in the endoscope information and inspect the leakage state of the tubular passages inside of the endoscope in a dry leakage test way.

For this, the endoscope 10 is equipped with an RFID tag 30 which is employed to send its unique identification information, namely, an RFID information (for example, a serial number, etc.) to the automatic endoscope leakage inspection apparatus 100. Moreover,the automatic endoscope leakage inspection apparatus 100 may be equipped with an RFID module 190 which is able to read out a unique identification information with respect to the mounted endoscope 30 from the RFID tag 30 when it is mounted at the endoscope 10.

The automatic endoscope leakage inspection apparatus 100 according to the present invention may automatically process the leakage inspection of the endoscope 100 based on the optimum inspection condition, for example, a pressurizing pressure, a pressurizing time, a volume value, a stabilization time, etc. which are set based on kinds of the endoscopes 10 in such a way to use the endoscope information set and stored inside by kinds of the endoscopes 10 in response to the unique identification information of the endoscope 10 read from the RFID tag 30.

More specifically, the endoscope 10 may be provided in various kinds, wherein the inside tubular passages have different length and/or thicknesses based on the manufacturers and use purposes. For this reason, the endoscope 10 may have the inside tubular passages having different volume values. This endoscope 10 may be categorized into an inspection purpose and a surgical operation purpose and may include a gastro endoscope, a colono endoscope, a broncho endoscope, an ERCP (Endoscopic Retrograde Cholangio-Pancreatoscopy) endoscope and an ultrasonic endoscope. As illustrated in Figure 1, the endoscopes 10 have different sizes of tubular passages, but they have similar configurations and structures.

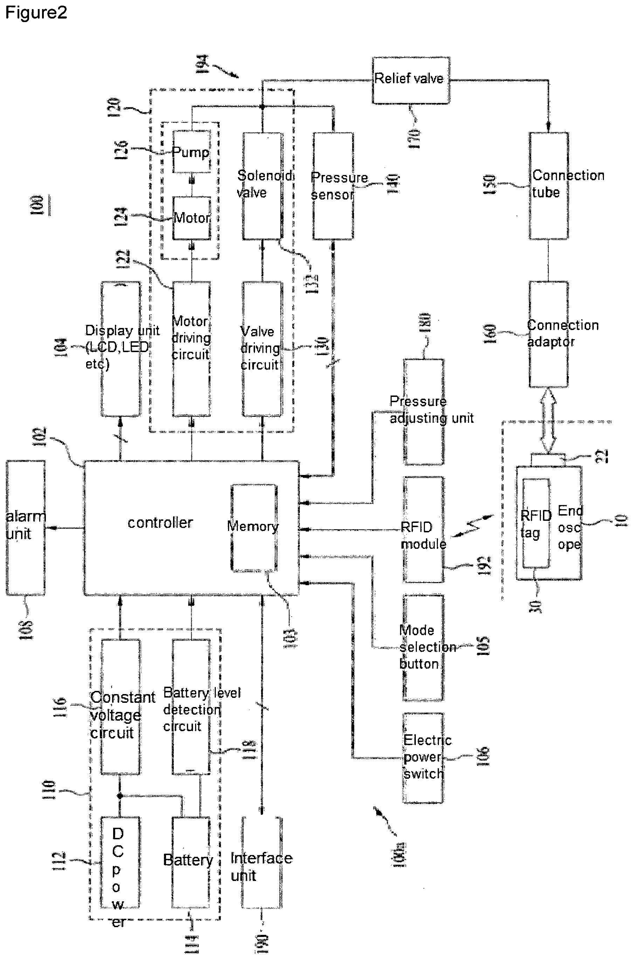

The endoscope 10 of the present embodiment may include an insertion unit 14, amanipulation unit 12, a universal unit 12 and a connector unit 20. Moreover, the endoscope 10 may equipped with an RFID tag (30 in Figure 2) which includes the serial number which may be different based on its unique identification information, for example, a manufacturer, a use purpose, etc.

The insertion unit 14 may be formed thin and long enough to be inserted into a body cavity. The front end of the insertion unit 14 may be provided with a scope unit 16 which is formed of a small-sized camera, for example, a CCD sensor. The endoscope 10 may be provided in the same or similar form as in Figure 1. For this reason, the detailed description on the endoscope 10 will be omitted. The endoscope 10 may have a configuration different based on various manufacturers and kinds of product models, for example, Olympus medical systems company, Fuzinon company, Pentax company, etc., but the automatic endoscope leakage inspection apparatus 100 according to the present invention is able to automatically inspect any leakage in such a way to directly or indirectly connect to the inside tubular passage of the endoscope 10.

The manipulation unit 12 may be connected to a rear end of the insertion unit 14 and is configured to rotate and bend in various directions the insertion unit 14 in order for a surgical operator to inspect or take a predetermined tissue. The universal cable 18 extends projecting from the manipulation unit 12, wherein a connector unit 20 is provided at an end of the universal cable 18. The connector cable 20 is provided with an air and water transfer connection port 22 and is connected with a peripheral device (not illustrated), for example, an air and water transfer device, a light source device, and a signal processing device. The connector unit 20 is connected with a peripheral device, thus emitting light or processing a video signal. Moreover, the connector unit 20 may be connected with the automatic endoscope leakage inspection device 100 via an air and water transfer connection port 22.

The automatic endoscope leakage inspection apparatus 100 according to the present invention is a portable device to automatically inspect any leakage of the endoscope, and it can be engaged at the connector unit 20 of the endoscope 10 or can be engaged in a separate form. More specifically, the automatic endoscope leakage inspection apparatus 100 may be mounted at the connector unit 20 to automatically inspect any leakage of the endoscope 10. This automatic endoscope leakage inspection apparatus 100 is able to supply the air of a predetermined pressure to the inside tubular passage (not illustrated) formed inside of the endoscope 10 and monitor any change in the pressure inside the endoscope 10, thus inspecting and determining the leakage of the endoscope.

The endoscope leakage inspection apparatus 100 according to the present invention can be engaged to or disengaged from the endoscope 10 and is provided in the form of a small size, which may allow to facilitate the portability and inspection. Since the leakage inspection is carried out in a dry leakage test manner, it is possible to quickly and accurately carry out the leakage inspection of the endoscope 10.

More specifically, the automatic endoscope leakage inspection apparatus 100 may include a connection adaptor 160 connected to the endoscope 10, a connection tube 150 engaged to the connection adaptor 160, and a tester 100a which is engaged with the connection tube 150, thus inspecting if any leakage occurs at the inside tubular passage of the endoscope 10.

The connection adaptor 160 may be fixedly inserted into the connector unit 20 of the endoscope 10 or may be disengaged therefrom and may be sealingly engaged with the connector unit 20, which is waterproofed.

The connection tube 150 may be fixedly engaged to the connection adaptor 160 and may be sealed. The connection tube 150 is made of, for example, a flexible material, wherein an end thereof is engaged at the connection adaptor 160, and the other end thereof is engaged at the tester 100a. This connection tube 150 is able to interconnect the endoscope 10 and the tester 100a via the connection adaptor 160.

Moreover, the tester 100a may include a housing 101 sized large enough for the sake of portability and storage, a display unit 104 provided at one surface of the housing 101, an electric power switch 106, and a plurality of mode selection buttons 105. In this embodiment, the mode selection button 105 is provided two in number, thus being able to select the automatic mode or the manual mode. The mode selection buttons 105 may be modified into various forms depending on the operation mode of the tester 100a. For example, it is obvious that the mode selection button 105 is provided one in number, thus selecting the automatic mode or the manual mode by turning on or off the mode selection button 105.

Moreover, one side of the housing 101 of the tester 100a may be provided with an electric power jack (or a socket) 109 into which an electric power plug is inserted to supply a DC power, and a connection port 107 for connecting the connection tube 150. The connection port 107 may be configured in such a way that the connection tube 150 is fixedly inserted into or is disengaged from the connection port 107.

The configuration of the automatic endoscope leakage inspection apparatus according to the present invention will be described with reference to Figures 2 to 9.

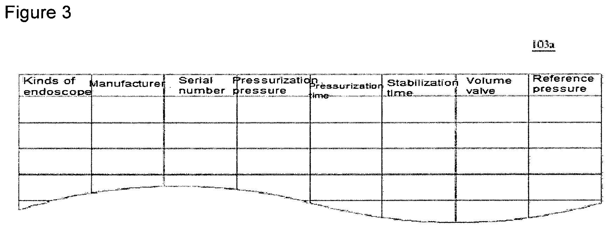

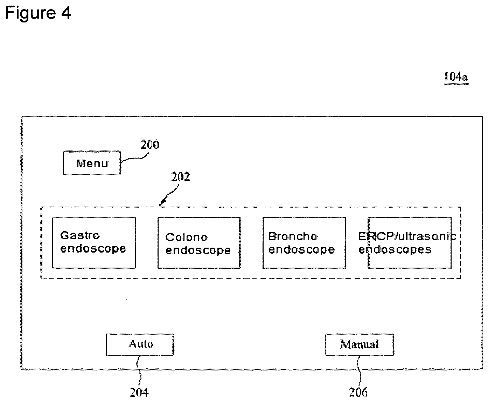

Figure 2 is a block diagram illustrating a configuration of an automatic endoscope leakage inspection apparatus in Figure 1. Figure 3 is a view illustrating a configuration of an endoscope information set in a memory in Figure 2. Figures 4 to 9 are views illustrating a setting procedure and an inspection result based on the kinds of the endoscopes are displayed on a display unit in Figure 1.

Referring to Figure 2, the automatic endoscope leakage inspection apparatus 100 may include an RFID module 192 to rear out the unique identification information with respect to the endoscope 10 from the RFID tag 30 provided at the endoscope 10. If the endoscope 10 is connected to the tester 100a, the RFID module 192 will read out the unique identification information from the RFID tag 30 of the endoscope 10 in the automatic mode.

The automatic endoscope leakage inspection apparatus 100 my include a controller 102 for controlling the whole operations, an electric power supply unit 110 for supplying a DC electric power, a display unit 104 for displaying various information related with the leakage inspection, operation state and inspection result of the tester 100a, an inspection module 194 configured to supply for a predetermined time the air with a pressurizing pressure set for the tubular passage inside the endoscope 10, an interface unit 190 connected to an external electronic device (for example, a computer device) (not illustrated) and configured to write or update a control program stored in the memory 103 provides inside of the controller 102, and an alarm unit 108 configured to output various audio signals based on an operation state and an inspection result of the tester 100a.

Moreover, the automatic endoscope leakage inspection apparatus 100 includes a plurality of mode selection buttons 105. The automatic endoscope leakage inspection apparatus 100 may further include a pressure adjusting unit 180 to variably adjust a constant pressure. The pressure adjusting unit 180 is equipped with a plurality of up and down buttons, thus adjusting the levels of the pressurization and measurement pressure of the tester 100a upwardly or downwardly. The automatic endoscope leakage inspection apparatus 100 in this embodiment may be set to pressurize on the inside tubular passage of the endoscope 10 via the inspection module 194 or adjust the pressure value measured by the pressure sensor 140 of the inspection module 194 in a pressure range of about 200∼300mmHg to the maximum; however it is able to adjust the range of the pressurization and measurement pressure value using the pressure adjusting unit 180 for the sake of being appropriate to various kinds of the endoscopes 10. For example, in case of the endoscope of the bronchus operation, the pressure of the tester 100a may be adjusted up to about 250mmHg to the maximum.

The controller 102 is equipped with a microprocessor, a microcontroller, etc. The controller 102 is also equipped with a writable and readable memory 103 and is configured to set and store, in the memory 103, the endoscope information (103a in Figure 3) based on various kinds of the endoscopes by unique identification information, and store the control program to process the whole operations of the tester 100a.

In this embodiment, as illustrated in Figure 3, the endoscope information 103a may contain kinds of various endoscopes, a manufacturer, a serial number, a pressurizing pressure, a pressurizing time, a stabilization time, a volume value and a reference input level.

The kinds of the endoscopes may set a gastro endoscope, a colono endoscope, a broncho endoscope, an ERCP endoscope and an ultrasonic endoscope. The serial number may contain the unique identification information contained in the RFID tag 30 provided at the endoscope 10. This serial number may be set different to various endoscopes.

The pressurizing pressure allows to set the maximum pressure value of the air which is supplied to the inside tubular passage in response to the inside tubular tube having different sizes of the endoscopes 10. The pressurizing time allows to set the leakage inspection time to determine the leakage inspection using the pressurizing pressure. The stabilization time allows to set the time that the air is supplied with the pressurizing pressure, and the pressure becomes the pressurizing pressure. Moreover, the reference pressure allows to set and store the error value of a permissible range to determine if the measured pressure value is an error in the permissible range.

The control program of the controller 102 may store the processing algorithm based on the endoscope leakage inspection mode of the tester 100a. For example, the endoscope leakage inspection mode may be formed of an automatic mode and a manual mode. If one endoscope leakage inspection mode is selected by the mode selection button 105, the controller 102 may inspect the leakage state of the endoscope 10 in any of the automatic mode wherein the leakage inspection is automatically carried out by reading out the unique identification information from the RFID tag 30 in response thereto and the manual mode wherein the leakage inspection is manually carried out by selecting the endoscope information via the same. Since this inspection method is described in the endoscope leakage inspection of the Korean patent registration number 10-1052893 (the laid-open data is July 29, 2011), the detailed description thereon will be omitted. For example, the manual mode may be available if a small quantity of the endoscopes is provided or if the worker knows the serial number of the endoscope.

The electric power supply unit 110 may include a DC power unit 112 for receiving the AC power and supplying AC power, a battery 114 disposed inside of the tester 100a and configured to supply the DC power if the DC power unit 112 is not connected, a constant voltage circuit 116 for receiving the DC power supplied from the DC power unit 112 and the battery 114 and supplying the same in the form of a constant voltage, and a battery level detection circuit 118 for detecting the residual level of the power of the battery 114. The DC power unit 110 may be provided, for example, in the form of an electric power adaptor having an electric power plug and may be inserted in the electric power provided at one side of the housing 101 of the tester 100a or may be provided, for example, in the form of the electric power supply circuit which is disposed inside of the tester 100a and receives the AC power and supplies the same in the form of the DC power. If the residual amount of the electric power of the battery 114 is detected low, the battery level detection circuit 118 supplies a detection result to the controller 102.

The inspection module 194 may include an air supply unit 120 for supplying the air of a constant pressure to the inside tubular passage of the endoscope 10, a pressure sensor 140 for measuring any change in the pressure at the inside tubular passage of the endoscope 10, and a relief valve 170 disposed between the connection tube 150 and the pump 126, thus maintaining a stable pressurization.

The air supply unit 120 may include a motor driving circuit 122, a motor 124 and a pump 126 which are provided so as to supply the air of a constant pressure to the inside tubular passage of the endoscope 10. The motor 124 and the pump 126 may be provided in an integrated form. The motor driving circuit 122 is able to drive the motor 124 in response to a control of the controller 102. The motor 124 may operate the pump 126 in response to a constant pressure (for example, about 300mmHg). The pump 126 may allow to supply the air of a constant pressure to the inside tubular passage of the endoscope 10 via the connection tube 160 and the connection adaptor 170.

The air supply unit 120 may include a valve driving circuit 130 and a solenoid valve 132 both for discharging the pressurized air from the inside tubular passage of the endoscope 10. The valve driving circuit 130 is able to open or close the solenoid valve 132 in response to a control of the controller 102. The solenoid valve 132 may be connected to the connection tube 160. If the leakage inspection is completed by the tester 100a, the valve driving circuit 130 opens the solenoid valve 132 and discharge the air supplied to the inside tubular passage of the endoscope 10. The valve driving circuit 132 may periodically open the solenoid valve 132 for a leakage inspection time for the sake of the repeated inspection in the automatic mode after the air of a constant pressure has been supplied to the inside tubular passage.

The pressure sensor 140 is able to measure the pressure in real time or periodically of the inside tubular passage of the endoscope 10. The pressure sensor 140 may be substituted with a sensor, a flow rate sensor, etc. which are able to measure any change to the flow rate of the air pressurized to the inside tubular passage of the endoscope 10. In this way, the pressure sensor 140 may measure the pressure of the inside tubular passage in response to a control signal from the controller 102 and convert a result of the measurement into an electric signal and output it to the controller 102.

As the leakage inspection is completed, the alarm unit 108 may output the normal state of the endoscope 10 or the leakage occurrence state in the form of an audio signal. For example, if the endoscope 10 is determined as a normal state as a result of the inspection, the alarm unit 108 may output an alarm sound once, and if the endoscope 10 is in a leakage state, it may output the alarm sound three times. Moreover, the alarm unit 108 may be configured to output another type of the alarm sound if the residual level of the battery is a low level.

The relief valve 170 is employed to protect the tester 100a and the endoscope 10 in such a way that the relief valve 170 is automatically opened and discharges air if the air the pressure value of which is over the maximum permissible pressure value, is supplied to the inside tubular passage of the endoscope 10, wherein the value of the pressure is pressurized by the pump 126 in case where an error occurs at the endoscope 10.

Moreover, the display unit 104 may be formed of a LCD device and is able to display various information. The display unit 104 may be further equipped with a plurality of light emitting diodes (LED).

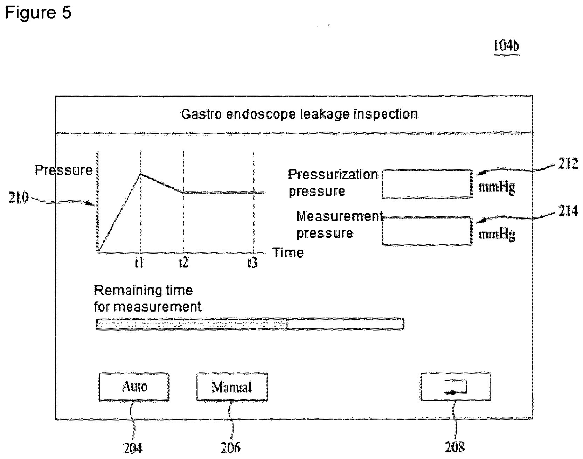

As illustrated in Figures 4 and 5, if the endoscope 10 is mounted on the tester 100a, this display unit 104 may allow to select the automatic mode 204 or the manual mode 206 to determine the kinds 202 of the endoscopes 10 on the initial screen 104a, and if one endoscope (for example, a gastro endoscope) is determined in the automatic mode from the RFID tag 30, the display unit 104 may display, on the endoscope recognition screen 104b configured to display the endoscope operation state, the endoscope information set for the sake of a leakage inspection with respect to a corresponding endoscope, namely, a pressurizing and stabilizing time 210, a pressurizing pressure level 212, a measurement pressure level 214 and the remaining time.

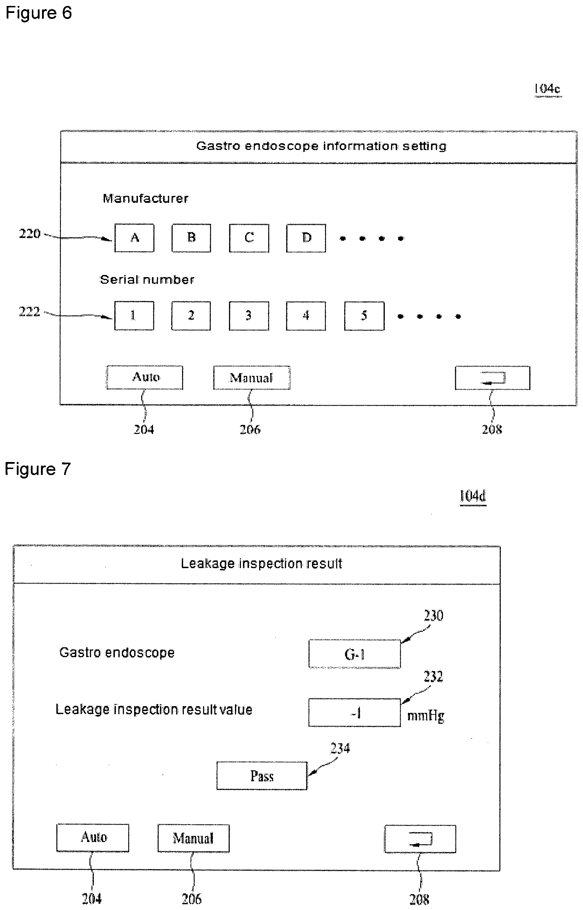

As illustrated in Figure 6, the display unit 104 may allow to input the information on the manufacturer 220 of the endoscope and the serial number 222 on the setting screen 104c configured to set the endoscope information with respect to each endoscope. Moreover, the display unit 104 may display the leakage inspection result display screens 104d to 104f which show a result of the leakage inspection. At this time, the leakage inspection result display screens 104d to 104f may be configured to display the kinds 230, 240 and 250 of the endoscopes, the leakage inspection result values 232, 242 and 252, namely, the inspection result pressure value, and the states showing that the inspection results are the state 234, the bad state 254 and the check-required state 244 based on the reference pressure value of the endoscope information.

The display unit 104 may display an electric power supply state, an error occurrence state, an inspection on-going state, a battery low level state, etc. using a plurality of the light emitting diodes (not illustrated).

As described above, the endoscope leakage inspection apparatus 100 according to the present invention is able to easily inspect the leakage occurrence state in such a way to read out a unique identification information with respect to the endoscope 10 from the RFID tag 30 disposed at the endoscope 10 using the RFID module 192 and pressurize the air of a constant pressure to the inside tubular passage of a corresponding endoscope 10 by determining the kinds of the endoscope via the same. The endoscope leakage inspection apparatus 100, therefore, is able to confirm a leakage portion of the endoscope in such a way to proceed with a leakage inspection using the automatic mode, recognize the leakage occurrence state of the endoscope 10, input the endoscope 10 equipped with the endoscope leakage inspection apparatus 100 if the leakage has occurred, into a processing tank (not illustrated) filled with water and then pressurize the air to the inside tubular passage of the endoscope using the manual mode.

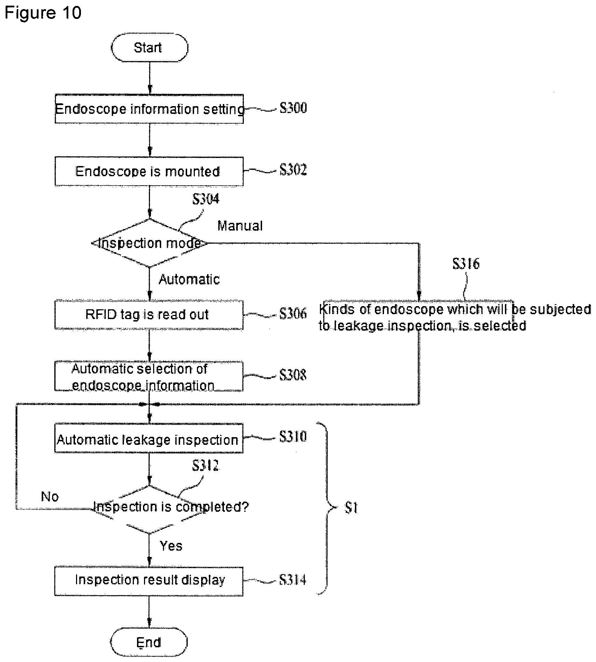

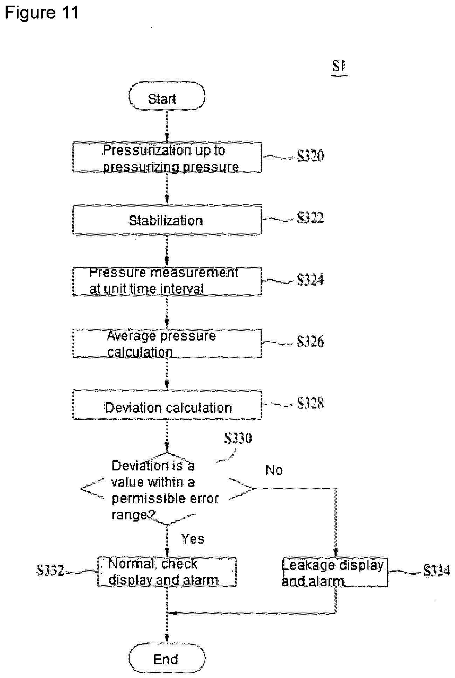

Continuously, Figure 10 is a flow chart illustrating an inspection procedure of an automatic endoscope leakage inspection apparatus according to the present invention, and Figure 11 is a flow chart illustrating an automatic leakage inspection procedure in Figure 10.

Referring to Figure 10, in a step S300, the endoscope information 103a with respect to each of a plurality of the endoscopes 10 are set and stored based on various kinds in the automatic endoscope leakage inspection apparatus 100. In this embodiment, as illustrated in Figure 3, the endoscope information 103a may contain the kind of an endoscope, a manufacturer, a serial number, a pressurizing pressure, a pressurizing time, a stabilization time, a volume value and a reference pressure.

In a step S302, if the endoscope 10 is mounted on the tester 100a, an inspection mode is selected to determine if the leakage inspection with respect to the endoscope 10 mounted in the step S304 is carried out in the automatic mode or the manual mode.

If the inspection mode is selected as the automatic mode, this procedure goes to the step S306, and the RFID tag 30 of the mounted endoscope 10 is read out using the RFID module 192, and in a step S308, the endoscope information corresponding to the unique identification information read out from the RFID tag 30 will be automatically selected.

If the inspection mode is selected as the manual mode, this procedure goes to the step S316, and the kind of the endoscope which will be subjected to the leakage inspection may be selected. At this time, if the kind of the endoscope is selected, the endoscope information based on the selected endoscope will be automatically selected. This procedure is appropriate if the kinds or a small quantity of the endoscopes are provided or the worker knows the kinds of the mounted endoscope or the serial number.

If the endoscope information is selected automatically or manually, the automatic leakage inspection is carried out in the steps S310 to S314. If the inspection is completed, a result of the inspection will be displayed. As illustrated in Figure 11, these steps (S1: S310 to S314) are referred to the processing of the automatic leakage inspection.

Referring to Figure 11, in a step S320, the air is pressurized to the inside tubular passage of the endoscope 10 up to a pressurizing pressure set at the controller 102. In a step S322, the inside of the tubular passage is stabilized for the inside thereof to reach a set pressure for a predetermined time period and to maintain the reached state. In a step S324, if the stabilization is obtained, the pressure of the inside tubular passage of the endoscope 10 is measured at a unit time interval via the pressure sensor 140.

The average pressure values of the pressures measured at the unit time interval in the step S326 are calculated, and any deviation with respect to the average pressure value is calculated in a step S280.

In a step S330, it is determined if the deviation calculated in the step S330 is within a permissible error range. This may be determined in such a way to compare to the deviation set and stored at the controller 102. As a result of the determination, if the calculated deviation is a valve within a permissible error range, this procedure will go to the step S332, and the normality or checked state will be displayed or reported through the display unit 104 and the alarm unit 108. If the calculated deviation is not a value within a permissible error range, the procedure will go to the step S334, and the leakage occurrence state will be displayed and reported through the display unit 104 and the alarm unit 108.

The endoscope leakage inspection method in the automatic mode may require about 30∼40 seconds for the pressurization, stabilization, inspection and determination. In this way, the leakage state of the endoscope 10 can be quickly inspected.

Since only the leakage occurrence state of the endoscope 10 is inspected in the automatic mode of the present invention, it is possible to quickly and easily recognize the leakage occurrence state in such a way to engage the endoscope 10 to the automatic endoscope leakage inspection apparatus 100 at any time before or after the surgical operation.

As the present invention may be embodied in several forms without departing from the spirit or essential characteristics thereof, it should also be understood that the above-described examples are not limited by any of the details of the foregoing description, unless otherwise specified, but rather should be construed broadly within its spirit and scope as defined in the appended claims, and therefore all changes and modifications that fall within the meets and bounds of the claims, or equivalences of such meets and bounds are therefore intended to be embraced by the appended claims.

## Claims

1. An automatic endoscope leakage inspection apparatus, comprising:
an RFID (Radio Frequency Identification) tag which is provided at an endoscope having an inside tubular passage and has a unique identification information on the endoscope;
an RFID module which is able to read out the unique identification information on the endoscope, which will be subjected to a leakage inspection, from the RFID tag;
an inspection module which is formed of an air supply unit configured to supply the air of a constant pressure to the inside tubular passage of the endoscope, a pressure sensor configured to measure a pressure change in the inside tubular passage of the endoscope, and a relief valve configured to maintain a stable pressurization on the inside tubular passage of the endoscope, wherein the inspection module is able to supply, for a predetermined time period, the air of a set pressurization pressure to the inside tubular passage of the endoscope and is able to detect the pressure change; and
a controller which is configured to set and store a plurality of endoscope information corresponding to the endoscopes of various kinds in the inside thereof, control for the RFID module to read out the unique identification information from the RFID tag, set a pressurization pressure, a pressurization time, a volume value and a stabilization time by kinds of the endoscopes in such a way to use the endoscope information corresponding to the unique identification information read out using the RFID module, supply the air of a constant pressure to the inside tubular passage formed inside of the endoscope, monitor a pressure change in the inside of the endoscope via the same, control the inspection module for the leakage of the inside tubular passage of the endoscope to be automatically inspected, and determine the leakage inspection state of the endoscope in response to the pressure change detected by the inspection module.

2. The apparatus of claim 1, wherein the automatic endoscope leakage inspection apparatus further comprises a display unit which is able to display a leakage inspection state of the endoscope determined by the controller.

3. The apparatus of claim 1, wherein the controller is able to manually select the endoscope information with respect to the endoscope which will be subjected to a leakage inspection.

4. An inspection method of an automatic endoscope leakage inspection apparatus which is able to automatically carry out a leakage inspection of the endoscope formed of an RFID tag including a unique identification information, comprising:
setting and storing an endoscope information with respect to each of a plurality of endoscopes based on various kinds thereof at the automatic endoscope leakage inspection apparatus;
selecting an inspection mode on if the leakage inspection is proceeded in the automatic mode or the manual mode with respect to an endoscope when the endoscope has been mounted on the automatic endoscope leakage inspection apparatus;
reading out the RFID tag of the mounted endoscope using an RFID module of the automatic endoscope leakage inspection apparatus when the inspection mode is selected as the automatic mode;
automatically selecting the endoscope information corresponding to the unique identification information read out from the RFID tag; and
setting a pressurization pressure, a pressurization time, a volume value and a stabilization time based on the kinds of the endoscopes in response to the endoscope information when the endoscope information is automatically selected, and pressurizing the air to an inside tubular passage of the endoscope, and carrying out an automatic leakage inspection.

5. The method of claim 4, wherein the inspection method is referred to manually selecting the kinds of the endoscope which will be subjected to the leakage inspection if the inspection mode is selected as the manual mode, and automatically selecting the endoscope information corresponding to the kinds of the selected endoscope.

6. The method of claim 5, wherein the inspection method further comprises displaying a result of the leakage inspection which has been carried out.
